# FASCICULE DE BREVET EUROPEEN

(11) **EP 1 025 833 B1**
(45) Date de publication et mention de la délivrance du brevet: **12.05.2004**
(21) Numéro de dépôt: 00400053.5
(22) Date de dépôt: 11.01.2000
(51) Int. Cl.: A61K 7/06

(54) **Compositions cosmétiques détergentes et utilisation de ces dernières**
Kosmetische Tensid-Präparate und deren Verwendung
Detergent cosmetic compositions and their use

(30) Priorité: 03.02.1999 FR 9901238
(43) Date de publication de la demande: 09.08.2000
(73) Titulaire: L'OREAL, 75008 Paris (FR)
(72) Inventeur: Cauwet-Martin, Danièle, 75011 Paris (FR); Restle, Serge, 95390 Saint Prix (FR)
(74) Mandataire: Le Blainvaux Bellegarde, Françoise

(56) Documents cités:
- DE-A- 4 233 385
- DE-A- 4 409 189
- DE-A- 19 723 763
- FR-A- 2 745 175
- FR-A- 2 745 176
- FR-A- 2 749 506
- FR-A- 2 756 488

## Description

La présente invention concerne de nouvelles compositions cosmétiques à propriétés améliorées destinées simultanément au nettoyage, au conditionnement et au coiffage des matières kératiniques telles que les cheveux, et comprenant, dans un support cosmétiquement acceptable, une base lavante constituée de tensio-actifs à pouvoir détergent dans laquelle sont également présents des polymères cationiques en association avec un polyuréthane ou un polyurée anionique soluble dans l'eau. L'invention concerne aussi l'utilisation desdites compositions dans l'application cosmétique susmentionnée.

Pour le nettoyage et/ou le lavage des cheveux, l'utilisation de compositions capillaires détergentes (ou shampooings) à base essentiellement d'agents tensio-actifs classiques de type notamment anioniques, non-ioniques et/ou amphothères, mais plus particulièrement de type anioniques, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entrainer à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abimés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, qui confèrent en effet aux cheveux lavés, secs ou mouillés, une facilité de démélage, une douceur et un lissage nettement accrus par rapport à ce qui peut être obtenu avec les compositions nettoyantes correspondantes qui en sont exemptes.

Par ailleurs, depuis quelque temps, on cherche à obtenir des shampooings conditionneurs qui soient capables d'apporter aux cheveux lavés non seulement les propriétés cosmétiques mentionnées ci-avant mais aussi, à un degré plus ou moins poussé, des propriétés de coiffage, de volume, de mise en forme et de tenue. Ces derniers shampooings lavants à propriétés cosmétiques générales améliorées sont souvent dénommés, par simplicité, "shampooings à effets coiffants", expression qui sera d'ailleurs reprise dans la suite de l'exposé.

Toutefois, et malgré les progrès réalisés récemment dans le domaine des shampooings à effets coiffants à base de polymères cationiques, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoqués ci-avant, de meilleures performances.

La présente invention vise à la satisfaction d'un tel besoin.

Ainsi, à la suite d'importantes recherches menées sur la question, il a maintenant été trouvé par la Demanderesse, de façon totalement inattendue et surprenante, qu'en introduisant un polycondensat anionique comprenant au moins une séquence polyuréthanne et/ou polyurée, tel que défini ci-après, dans des compositions détergentes en particulier capillaires contenant des polymères cationiques, il est possible d'améliorer de manière substantielle et significative les propriétés cosmétiques attachées à ces dernières, et ceci tout en conservant leur bon pouvoir lavant intrinsèque.

Dans la description et les revendications, le terme polyuréthane sera employé comme équivalent à polycondensat comprenant au moins une séquence polyuréthanne et/ou polyurée.

Sans vouloir limiter la présente invention à une quelconque théorie, il semblerait exister lors du rinçage, des interactions et/ou des affinités particulières entre les polymères cationiques, les polyuréthanes anioniques conformes à l'invention et les cheveux, qui favorisent un dépôt régulier, important et durable desdits polyuréthanes et polymères cationiques à la surface desdits cheveux, ce dépôt qualitatif et quantitatif étant probablement l'une des causes de l'amélioration observée au niveau des propriétés finales, en particulier la facilité de coiffage, le maintien, la nervosité et le volume des cheveux traités. En tout état de cause, les propriétés cosmétiques attachées aux bases lavantes contenant l'association d'agents [polymère cationique/polyuréthane] conforme à l'invention sont nettement supérieures à celles qui peuvent être obtenues en ne mettant en oeuvre qu'un seul de ces agents à concentration globale équivalente.

Toutes ces découverte sont à la base la présente invention.

Ainsi, selon la présente invention, il est maintenant proposé de nouvelles compositions détergentes en particulier capillaires, comprenant, dans un milieu cosmétiquement acceptable, une base lavante, au moins un polymère cationique, et au moins un polycondensat anionique comprenant au moins une séquence polyuréthanne et/ou polyurée hydrosoluble

L'invention a également pour objet l'utilisation en cosmétique des compositions ci-dessus pour le nettoyage, le conditionnement et le coiffage des matières kératiniques en particulier les cheveux.

Mais d'autres caractéristiques, aspects et avantages de l'invention apparaîtront encore plus clairement à la lecture de la description qui va suivre ainsi que des exemples concrets, mais nullement limitatifs, destinés à l'illustrer.

Comme indiqué précédemment, les constituants essentiels rentrant dans la composition des produits capillaires de l'invention sont (i) le ou les tensioactifs à pouvoir détergent destinés à former la base lavante, (ii) le ou les polymères cationiques et (iii) le ou les polyuréthanes anioniques hydrosolubles.

### A- BASE LAVANTE:

Les compositions conformes à l'invention comprennent nécessairement une base lavante, généralement aqueuse.

Le ou les tensioactifs formant la base lavante peuvent être indifféremment choisis, seuls ou en mélanges, au sein des tensioactifs anioniques, amphotères, non ioniques, zwittérioniques et cationiques. Toutefois, selon l'invention, la base lavante comprend de préférence des tensioactifs anioniques ou des mélanges de tensioactifs anioniques et de tensioactifs amphotères ou de tensioactifs non ioniques.

La quantité minimale de base lavante est celle juste suffisante pour conférer à la composition finale un pouvoir moussant et/ou détergent satisfaisant, et des quantités trop importantes de base lavante n'apportent pas vraiment d'avantages supplémentaires.

Ainsi, selon l'invention, la base lavante peut représenter de 4 % à 50 % en poids, de préférence de 6 % à 25 % en poids, et encore plus préférentiellement de 8 % à 20 % en poids, du poids total de la composition finale.

Les tensioactifs convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates : les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates, les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

Les tensioactifs anioniques comportant un groupement carboxyliques sont particulièrement préférés.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO⁻) (2)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.

Dans les compositions conformes à l'invention, on utilise de préférence des mélanges d'agents tensioactifs et en particulier des mélanges d'agents tensioactifs anioniques et d'agents tensioactifs amphotères ou non ioniques. Un mélange particulièrement préféré est un mélange constitué d'au moins un agent tensioactif anionique carboxylique et d'au moins un agent tensioactif amphotère ou non ionique.

On utilise de préférence un agent tensioactif anionique choisi parmi les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leur mélange avec un tensioactif sulfoné ou sulfatés tels les alkyl(C₁₂-C₁₄) sulfates de sodium, de triéthanolamine ou d'ammonium, les alkyl (C₁₂-C₁₄)éthersulfates de sodium oxyéthylénés à 2,2 moles d'oxyde d'éthylène, le cocoyl iséthionate de sodium et leurs mélange avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL® C2M CONC" en solution aqueuse à 38 % de matière active ou sous la dénomination MIRANOL® C32;
- soit un agent tensioactif amphotère de type zwittérionique tel que les alkylbétaïnes en particulier la cocoylbétaïne commercialisée sous la dénomination "DEHYTON® AB 30" en solution aqueuse à 32 % de MA par la société HENKEL ou les alkylamidoalkylbétaïnes telles que la TEGOBETAINE® F50 commercialisée par la société GOLDSCHMIDT.
- soit un agent tensioactif non ionique de type alkylpoluglucoside.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.
On notera que les tensioactifs cationiques, dont l'utilisation n'est pas exclue, ne constituent pas des tensioactifs préférés pour la mise en oeuvre de la présente invention.

### B- POLYMERE(S) CATIONIQUE(S) :

Les compositions conformes à l'invention comprennent en outre nécessairement un polymère cationique.

Les agents conditionneurs de type polymères cationiques utilisables conformément à la présente invention peuvent être choisis parmi tous ceux déja connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519 863.

Les agents conditionneurs de type polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 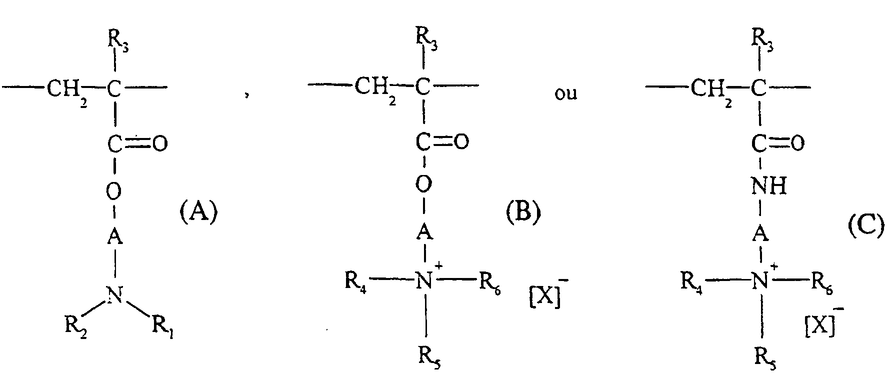 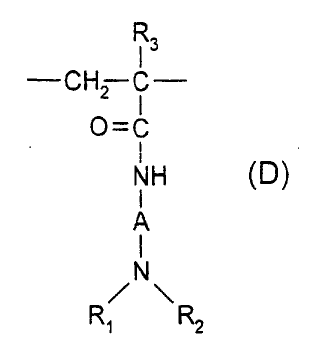 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle;
   X désigne un anion dérivé d'un acide minéral ou organique tel que un anion méthosulfate ou un halogénure tel que chlorure ou bromure.

   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP.
   - et les copolymères vinylpyrrolidone / méthacrylamide de diméthylaminopropyle quaternisé tel que le produit vendu sous la dénomination "GAFQUAT HS 100" par la société ISP.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d' épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) les cyclopolymères d'alkyl diallyl amine ou de dialkyl diallyl ammonium tels que les homopolymères ou copolymères comportant comme constituant principal de la chaîne des motifs répondant aux formules (VI) ou (VI') : 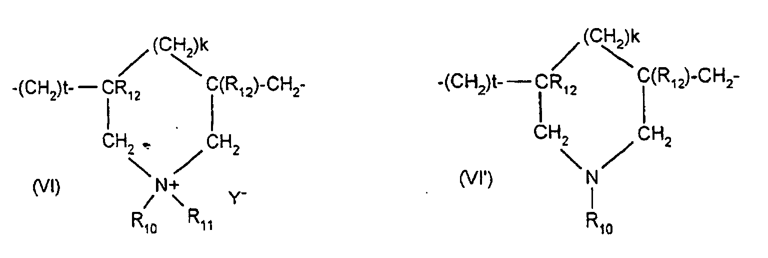 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faibles masses moléculaires moyenne en poids) et les copolymères de chlorure de diallyldiméthylammonium et d'acrylamide commercialisés sous la dénomination "MERQUAT 550".
(10) le polymère de diammonium quaternaire contenant des motifs récurrents répondant à la formule : 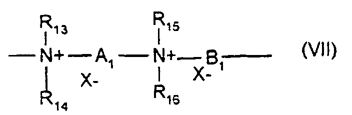
   formule (VII) dans laquelle :
   R13, R14, R15 et R16, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles contenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2-

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      - CH2-CH2-S-S-CH2-CH2- ;
   d) un groupement uréylène de formule : -NH-CO-NH- ;

   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 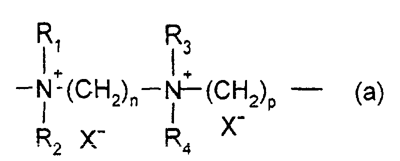 dans laquelle R₁, R₂, R₃ et R_{4,} identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (a) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).
(11) les polymères de polyammonium quaternaires constitués de motifs de formule (VIII): 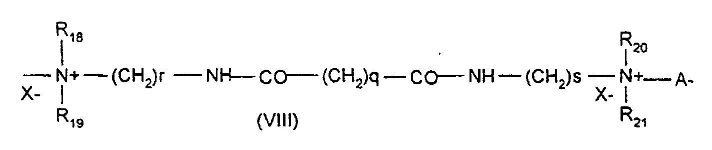 formule dans laquelle :
   R18, R19, R20 et R21, identiques ou différents, représentent un atome d'hydrogène ou un radical méthyle, éthyle, propyle, β-hydroxyéthyle, β-hydroxypropyle ou -CH2CH2(OCH2CH2)pOH,
   où p est égal à 0 ou à un nombre entier compris entre 1 et 6, sous réserve que R18, R19, R20 et R21 ne représentent pas simultanément un atome d'hydrogène,
   r et s, identiques ou différents, sont des nombres entiers compris entre 1 et 6,
   q est égal à 0 ou à un nombre entier compris entre 1 et 34,
   X désigne un atome d'halogène,
   A désigne un radical d'un dihalogénure ou représente de préférence -CH2-CH2-O-CH2-CH2-.
   De tels composés sont notamment décrits dans la demande de brevet EP-A-122 324.
   On peut par exemple citer parmi ceux-ci, les produits "Mirapol A 15", "Mirapol AD1", "Mirapol AZ1" et "Mirapol 175" vendus par la société Miranol.
(12) Les polymères quaternaires de vinylpyrrolidone et de vinylimidazole tels que par exemple les produits commercialisés sous les dénominations Luviquat FC 905, FC 550 et FC 370 par la société B.A.S.F.
(13) Les polyamines comme le Polyquart H vendu par HENKEL, référencé sous le nom de « POLYETHYLENEGLYCOL (15) TALLOW POLYAMINE » dans le dictionnaire CTFA.
(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société UNION CARBIDE CORPORATION, les cyclopolymères, en particulier les polymères ou copolymères de chlorure de diméthyldiallylammonium et d'acrylamide, vendus sous les dénominations « MERQUAT 100 », « MERQUAT 550 » et « MERQUAT S » par la Société CALGON, les polysaccharides cationiques tels que les gommes de guar modifiées par du chlorure de 2,3-époxypropyl triméthylammonium commercialisées par exemple sous la dénomination « JAGUAR C13S » par la société MEYHALL et les copolymère vinylpyrrolidone / méthacrylamidopropyl dimethylamine commercialisés notamment sous la dénomination STYLEZE CC 10 par la société ISP et leurs mélanges.

Selon l'invention, le ou les polymères cationiques peuvent représenter de 0,01 % à 10 % en poids, de préférence de 0,05 % à 5 % en poids, et encore plus préférentiellement de 0,1 % à 3 % en poids, du poids total de la composition finale.

### C- Polyuréthanes et/ou polyurées :

Selon une caractéristique essentielle des compositions capillaires détergentes conformes à l'invention, ces dernières contiennent en outre au moins un polycondensat comprenant au moins une séquence polyuréthanne et/ou polyurée, ledit polycondensat étant soluble dans l'eau.

Les polycondensats comprenant au moins une séquence polyuréthanne et/ou polyurée particulièrement visés par la présente invention sont ceux décrits dans les brevets EP 0 751 162, EP 0 637 600, FR 2 743 297 et EP 0 648 485 dont la Demanderesse est Titulaire, ainsi que le brevets EP 0 656 021 ou WO 94/03510 de la Société BASF et EP 0 619 111 de la Société National Starch.

Les polycondensats utilisés conformément à l'invention sont solubles dans l'eau, soit sous la forme acide, soit après neutralisation partielle ou totale par une base organique ou minérale.

Les polymères solubles dans l'eau selon la présente invention sont ceux qui conduisent en solution dans l'eau à des compositions limpides à l'oeil nu (à une concentration au moins égale à 1 % de matière active et à température ambiante (20-25°C)).

A titre d'exemple, le polycondensat peut être formé par un arrangement de blocs, cet arrangement étant obtenu notamment à partir de:
(1) au moins un composé qui contient deux ou plus de deux atomes d'hydro gène actifs par molécule;
(2) au moins un diol ou un mélange de diols contenant des radicaux acides ou leurs sels;
(3) au moins un di- ou polyisocyanate.

Avantageusement, les composés (1) sont choisis dans le groupe comprenant les diols, les diamines, les polyesterols, les polyétherols ou leur mélange.

Les composés (1) qui sont préférés sont les polyéthylène et les polypropylène glycols linéaires, en particulier ceux qui sont obtenus par réaction de l'oxyde d'éthylène ou de propylène avec l'eau ou du diéthylène ou du dipropylène glycol en présence d'hydroxyde de sodium en tant que catalyseur. Ces polyglycols ont généralement un poids moléculaire compris entre environ 600 et 20000.

D'autres composés organiques préférés sont ceux qui ont des groupes mercapto, amino, carboxyle ou hydroxyle. Parmi ceux-ci, on cite plus particulièrement les composés polyhydroxy tels que les polyéther diols, les polyester diols, les polyacétal diols, les polyamide diols, les polyester polyamide diols, les poly(alkylène éther) diols, les polythioéther diols et les polycarbonate diols.

Les polyéther diols préférés sont, par exemple, les produits de condensation d'oxyde d'éthylène, d'oxyde de propylène ou de tétrahydrofurane, leurs produits de copolymérisation ou de condensation, greffés ou blocs, tels que les mélanges de condensats d'oxyde d'éthylène et de propylène, et les produits de polymérisation d'oléfines, sous haute pression, avec les condensats d'oxyde d'alkylène. Des polyéthers appropriés sont par exemple préparés par condensation d'oxydes d'alkylène et d'alcools polyhydriques, tels que l'éthylène glycol, le 1,2-propylène glycol et le 1,4-butanediol.

Les polyesters diols, polyesters amides, polyamides diols sont de préférence saturés et sont obtenus, par exemple, à partir de la réaction d'acides polycarboxyliques saturés ou insaturés avec des alcools polyhydriques, des diamines ou des polyamines. Pour préparer ces composés, on peut utiliser, par exemple, l'acide adipique, l'acide succinique, l'acide phtalique, l'acide isophtalique, l'acide téréphtalique et l'acide maléique. Des alcools polyhydriques appropriés pour préparer les polyesters incluent par exemple l'éthylène glycol, le diéthylèneglycol, !e 1,2-propylène glycol, le 1,4-butanediol, le néopentyl glycol et l'hexane diol. On peut aussi utiliser des aminoalcools, par exemple l'éthanolamine. Des diamines appropriés pour préparer les amides polyesters sont l'éthylène diamine et l'hexaméthylène diamine.

Des polyacétals appropriés peuvent être préparés, par exemple, à partir de 1,4-butanediol ou d'hexanediol et de formaldéhyde. Des polythioéthers appropriés peuvent être préparés par exemple par réaction de condensation entre des thioglycol soit seuls ou en combinaison avec d'autres glycols tels que l'éthylène glycol, le 1,2-propylène glycol ou avec d'autres composés polyhydroxylés. Les composés polyhydroxylés contenant déjà des groupements urée ou uréthanne, des polyols naturels, qui peuvent être davantage modifiés, par exemple, l'huile de ricin et les carbohydrates peuvent également être utilisés.

Plus préférentiellement, le composé du groupe (1) est un polyestérol, notamment un polyester diol formé par la réaction d'au moins un (di)-polyol (1ₐ) et d'au moins un acide (1_{b}). Le (di)- polyol (1ₐ) est en particulier choisi dans le groupe comprenant le néopentylglycol, le butanediol-1,4, l'hexanediol, l'éthylèneglycol, le diéthylène glycol, le propylèneglycol, le butylèneglycol, le néopentylglycol et (di)-polyéthylèneglycol. L'acide (1_{b}) est en particulier choisi dans le groupe comprenant l'acide phtalique, l'acide isophtalique, l'acide adipique et l'acide (poly)-lactique.

En tant que composé (2), on peut notamment utiliser un acide hydroxycarboxylique tel que l'acide diméthylol propanoïque (DMPA) ou un acide carboxylique 2,2-hydroxyméthyl. En général, le composé (2) est utile en tant que bloc de couplage.

Les composés (2) particulièrement préférés conformément à l'invention sont ceux choisis dans le groupe comprenant le 2,2-di-(hydroxyméthyl) acide acétique, le 2,2-dihydroxyméthyl acide propionique, le 2,2-dihydroxyméthyl acide butyrique, le 2,2-dihydroxyméthyl acide pentanoïque.

Le di- ou polyisocyanate (3) peut être choisi en particulier dans le groupe comprenant l'hexaméthylène diisocyanate, l'isophoronediisocyanate (IPDI), le toluylènediisocyanate, le diphénylméthane 4,4'-diisocyanate (DPMD) et le dicyclohexylméthane 4,4'-diisocyanate (DCMD), le méthylène-di-p-phényl diisocyanate, le méthylène-bis(4-cyclohexylisocyanate), le toluène diisocyanate, le 1,5-naphtalène diisocyanate, le 4,4'-diphénylméthane diisocyanate, le 2,2'-diméthyl-4,4'-diphénylméthane diisocyanate, le 1,3-phénylène diisocyanate, le 1,4-phénylène diisocyanate, des mélanges de 2,4- et de 2,6- toluène diisocyanate, le 2,2'-dichloro-4,4'-diisocyanato diphénylméthane, le 2,4-dibromo-1,5-diisocyanato naphtalène, le 1,4-diisocyanate butane, l'hexane-1,6-diisocyanate, le cyclohexane-1,4-diisocyanate.

Le polycondensat peut être formé à l'aide d'un composé supplémentaire (4) servant en général à allonger la chaîne du polycondensat. Ces composés (4) peuvent être choisis dans la groupe comprenant notamment les glycols saturés ou insaturés tel que l'éthylène glycol, le diéthylène glycol, le néopentylglycol, le triéthylène glycol, les aminoalcools tels que l'éthanolamine, la propanolamine, la butanolamine, les amines primaires hétérocyclique, aromatique, cycloaliphatique, et aliphatique, les diamines, les acides carboxylique tels que les acides carboxyliques aliphatique, aromatique, hétérocyclique comme l'acide oxalique, succinique, glutarique, adipique, sébacique, téréphtalique, les acides aminocarboxyliques. Les composés (4) préférés sont les diols aliphatiques.

Un polyuréthane anionique particulièrement préféré est le polyurethane-1 (nom INCl) commercialisé par BASF sous la dénomination LUVISET PUR.

Les polycondensats conformes à l'invention peuvent également être formés à partir de composés supplémentaires (5) ayant un squelette siliconé tels que les polysiloxanes, les polyalkylsiloxanes ou les polyarylsiloxanes notamment les polyéthylsiloxanes, les polyméthylsiloxanes et les polyphénylsiloxanes, comportant éventuellement des chaînes hydrocarbonées greffées sur les atomes de silicium.

Les séquences de polyuréthanne et/ou polyurée du polymère utilisées avantageusement présentent un motif répétitif de base répondant à la formule générale ci-après:

- X' - B - X' - CO - NH - R - NH - CO - (I')

dans laquelle :
- X' représente O et/ou NH,
- B est un radical hydrocarboné bivalent, ce radical étant substitué ou non, et
- R est un radical divalent choisi parmi les radicaux alkylène de type aromatique, aliphatique en C₁ à C₂₀, cycloaliphatique en C₁ à C₂₀, ces radicaux étant substitués ou non.

De préférence, le radical B est un radical en C₁ à C₃₀ et est porteur d'un groupement présentant une ou des fonction(s) carboxylique(s) et/ou une ou des fonctions sulfoniques, lesdites fonctions carboxyliques et/ou sulfoniques étant sous forme libre ou bien neutralisées partiellement ou totalement par une base minérale ou organique.

Le radical R est avantageusement choisi parmi les radicaux répondant aux formules suivantes: 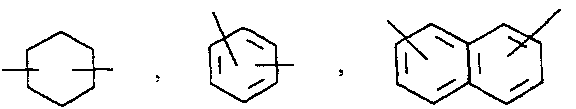 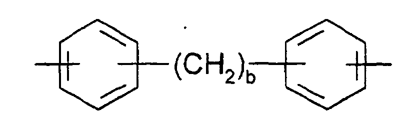 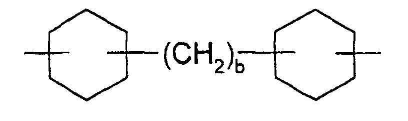 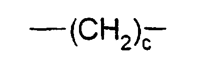 dans lesquelles b est un nombre entier compris entre 0 et 3, et c un nombre entier compris entre 1 et 20, de préférence compris entre 2 et 12.

En particulier, le radical R est choisi parmi les radicaux hexaméthylène, 4,4'-biphénylèneméthane, 2,4- et/ou 2,6-tolylène, 1,5-naphtylène, p-phénylène, méthylène-4,4bis - cyclohexyle et le radical divalent dérivé de l'isophorone.

Le polycondensat mis en oeuvre conformément à l'invention comprenant au moins une séquence polyuréthanne et/ou polyurée peut avantageusement comprendre en outre au moins une séquence polysiloxane dont le motif répétitif de base répond par exemple à la formule générale (II') ci-après:

- X' - P - X' - CO - NH - R - NH - CO - (II')

dans laquelle :
- P est un segment polysiloxanique,
- X' représente O et/ou NH, et
- R est un radical divalent choisi parmi les radicaux alkylènes de type aromatique, aliphatique en C₁ à C₂₀, cycloaliphatique en C₁ à C₂₀, ces radicaux étant substitués ou non.

Avantageusement, le segment polysiloxanique P répond à la formule générale ci-après: 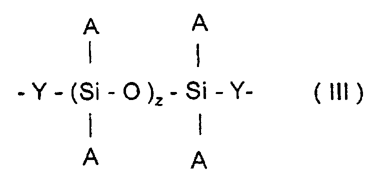 dans laquelle:
- les radicaux A, qui peuvent être identiques ou différents, sont choisis parmi d'une part les radicaux hydrocarbonés monovalents en C₁ à C₂₀ exempts ou substantiellement exempts d'insaturation éthylénique et, d'autre part, les radicaux aromatiques,
- Y représente un radical hydrocarboné bivalent, et
- z représente un nombre entier, choisi de telle sorte que le poids moléculaire moyen du segment polysiloxane soit compris entre 300 et 10 000.

En général, le radical bivalent Y est choisi parmi les radicaux alkylène de formule -(CH₂)ₐ- , dans laquelle a représente un nombre entier pouvant être compris entre 1 et 10.

Les radicaux A peuvent être choisis parmi les radicaux alkyles, en particulier les radicaux méthyle, éthyle, propyle, isopropyle, butyle, pentyle, hexyle, octyle, décyle, dodécyle et octadécyle, les radicaux cycloalkyle, en particulier le radical cyclohexyle, les radicaux aryle, notamment phényle et naphtyle, les radicaux arylalkyle, notamment benzyle et phényléthyle, ainsi que les radicaux tolyle et xylyle.

Les compositions capillaires conformes à l'invention renferment les polyuréthanes (polycondensats) définis ci-dessus à des teneurs pondérales qui peuvent être comprises entre 0,05 % et 10 %, de préférence entre 0,1 % et 5 % par rapport au poids total de la composition.

Généralement, le rapport Polymère cationique/Polyuréthane est compris entre 0,001 et 200 et de préférence entre 0,02 et 50 et encore plus particulièrement entre 0,1 et 20.

Le milieu aqueux cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.

Les compositions détergentes selon l'invention présentent un pH final généralement compris entre 3 et 10. De préférence, ce pH est compris entre 4 et 9. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les alcools gras supérieurs à C₁₆, les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions détergentes selon l'invention peuvent bien entendu contenir en outre tous les adjuvants usuels rencontrés dans le domaine des shampooings, comme par exemple des parfums, des agents conservateurs, des sequestrants, des épaississants, des adoucissants, des modificateurs de mousse, des colorants, des agents nacrants, des agents hydratants, des agents anti-pelliculaires ou anti-séborrhéiques, des filtres solaires et autres.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les agents tensioactifs cationiques, les polymères anioniques ou non ioniques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, le panthénol, les silicones, volatiles ou non volatiles, solubles et insolubles dans le milieu, les huiles végétales, les huiles de synthèses et leurs mélanges.

Les compositions selon l'invention peuvent contenir également des synergistes de mousses tels que des 1,2-alcanediols en C₁₀-C₁₈ ou des alcanolamides gras dérivés de mono ou de diéthanolamine.

Bien entendu, l'homme du métier veillera à choisir ce ou ces éventuels composés complémentaires et/ou leurs quantités de manière telle que les propriétés avantageuses attachées intrinsèquement à l'association (base lavante + Polymère cationique + polyuréthane selon l'invention) conforme à l'invention ne soient pas, ou substantiellement pas, altérées par la ou les adjonctions envisagées.

Ces compositions peuvent se présenter sous la forme de liquides plus ou moins épaissis, de crèmes ou de gel et elles conviennent principalement au lavage, au soin et/ou le coiffage des cheveux.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

Comme indiqué précédemment, les compositions conformes à l'invention confèrent aux cheveux, après rinçage, un remarquable effet coiffant qui se manifeste notamment par une facilité de coiffage et de maintien, ainsi qu'un apport de volume et de légèreté, nettement améliorés.

L'invention a également pour objet l'utilisation d'un polycondensat tel que defini ci-dessus pour ameliorer l'effet coiffant d'une composition capillaire détergente comprenant au moins un polymère cationique.

Des exemples concrets, mais nullement limitatifs, illustrant l'invention vont maintenant être donnés.

Dans les exemples, les composés suivants sont utilisés :
LUVISET PUR (BASF)
   Polyurethane-1 (nom INCI) en solution hydroalcoolique à 30% de matière active Polyuréthane soluble dans l'eau
AVALURE UR 450 (GOODRICH)
   Copolymère propylène glycol / isophorone diisocyanate / acide diméthylolpropanoïque en dispersion aqueuse à 38% de matière active. Polyuréthane insoluble dans l'eau
AKYPOSOFT 45 NV (KAO)
   Lauryl éther carboxylate de sodium à 4,5 moles d'oxyde d'éthylène en solution aqueuse à 22% de matière active
AG 10 LK (KAO)
   Alkyl (C₉/C₁₁) polyglucoside (1.4) en solution aqueuse à 40% de matière active
282930 (NATIONAL STARCH)
   Terpolymère acétate de vinyle / acide crotonique / néodécanoate de vinyle
STYLEZE CC 10 (ISP)
   Copolymère vinylpyrrolidone / méthacrylamidopropyl diméthylamine (80/20 en poids) en solution aqueuse à 10% de matière active
MERQUAT 100 (CALGON)
   Homopolymère de chlorure de diallyldiméthylammonium en solution aqueuse à 40% de matière active
MERQUAT 550 (CALGON)
   Copolymère chlorure de diallyl diméthyl ammonium / acrylamide 50/50 (en poids) en solution aqueuse à 8 % de matière active.
JAGUAR C 13 S (RHODIA CHIMIE)
   Chlorure d'hydroxypropylguar triméthyl ammonium.
DEHYTON AB 30 (HENKEL)
   Cocoyl bétaïne en solution aqueuse à 30% de matière active.

### EXEMPLE 1

On a réalisé deux compositions de shampooing, l'une conforme à l'invention (composition A) et l'autre comparative (composition B), différant l'une de l'autre simplement par la nature du polymère anionique employé :

| | **A(invention)** | **B** |
|---|---|---|
| AKYPOSOFT 45 NV (KAO) | 12 g MA | 12 g MA |
| AG 10 LK (KAO) | 2 g MA | 2 g MA |
| NaCl | 2 g | 2 g |
| STYLEZE CC 10 (ISP) | 1 g MA | 1 g MA |
| LUVISET PUR (BASF) | 3 g MA | ₋ |
| 282930 (NATIONAL STARCH) | ₋ | 3 g MA |
| | | |
| Eau qsp | 100 g | 100 g |
| pH ajusté à | 6.9 | 6.9 |
| | | |
| Aspect des compositions | transparente | trouble |

Des mèches de 2,5 g de cheveux naturels d'une longueur de 27 cm sont préalablement humidifiées puis mises en contact avec 1 g de la composition A selon l'invention pendant 5 minutes puis rincées à l'eau. Les mèches sont ensuite séchées sous un casque pendant 20 minutes à 65°C. On procède selon le même mode opératoire que ci-dessus avec la composition comparative B.

### Evaluation du toucher des mèches.

On présente à un panel de 10 testeurs les mèches préparées comme indiqué ci-dessus.

On leur demande d'indiquer les mèches qu'ils jugent plus douces et lisses.

Les 10 juges sont unanimes et déclarent que les cheveux traités à l'aide de la composition A sont plus doux et lisses.

### Evaluation des propriétés fixantes

On entend par propriétés fixantes la cohésion apportée à un ensemble de cheveux par le dépôt d'un matériau limitant leur déplacement relatif ainsi que la stabilité dans le temps de l'ensemble de cheveux formé.

Un moyen simple d'appréhender ces propriétés consiste à étudier la détente de mèches.

Pour cela, des mèches de 2,5 g de cheveux naturels d'une longueur L₀ de 27 cm sont préalablement humidifiées puis mises en contact avec 1 g de la composition A selon l'invention pendant 5 minutes puis rincées à l'eau. Les mèches encore humides sont ensuite enroulées sur des bigoudis de 2 cm de diamètre et de 7 cm de long. Les mèches sont ensuite séchées 20 minutes au casque-chauffant. Après séchage, les mèches sont dégagées des bigoudis avec soin et suspendues par leur attache à un support fixe de telle sorte que les cheveux pendent librement sous leur propre poids.

On procède selon le même mode opératoire que ci-dessus avec la composition comparative B.

On mesure alors la longueur (L) des mèches bouclées suspendues par leur seul poids devant un panneau gradué.

On les laisse ensuite se relaxer, toujours à l'état suspendu, pendant 2 heures à température ambiante.

La longueur des mèches suspendues s'accroît alors d'une certaine longueur (ΔL). Plus cet allongement ΔL est faible, meilleure est la tenue du coiffage dans le temps.

Ainsi, avec la composition B, l'allongement était de 3 cm (moyenne de 2 mèches), alors qu'il n'était que de 2,55 cm (moyenne de 2 mèches) pour la composition A, soit une amélioration dans la réduction de l'allongement de plus de 15%, ce qui traduit bien la meilleure tenue de coiffage obtenue grâce au shampooing selon l'invention.

### EXEMPLE 2

On a réalisé deux compositions de shampooing, l'une conforme à l'invention (composition A) et l'autre comparative (composition C), différant l'une de l'autre simplement par la solubilité du polyuréthane anionique employé :

| | **A(Invention)** | **C** |
|---|---|---|
| AKYPOSOFT 45 NV (KAO) | 12 g MA | 12 g MA |
| AG 10 LK (KAO) | 2 g MA | 2 g MA |
| NaCl | 2 g | 2 g |
| STYLEZE CC 10 (ISP) | 1 g MA | 1 g MA |
| LUVISET PUR (BASF) | 3 g MA | ₋ |
| AVALURE UR 450∗ (GOODRICH) | ₋ | 3 g MA |
| | | |
| Eau qsp | 100 g | 100 g |
| pH ajusté à | 6.9 | 6.9 |
| | | |
| Aspect des compositions | transparente | trouble |
| L'AVALURE UR 450 est un polyuréthane anionique insoluble dans l'eau. | | |

Des mèches de 2,5 g de cheveux naturels d'une longueur de 27 cm sont préalablement humidifiées puis mises en contact avec 1 g de la composition A selon l'invention pendant 5 minutes puis rincées à l'eau. Les mèches sont ensuite séchées sous un casque pendant 20 minutes à 65°C. On procède selon le même mode opératoire que ci-dessus avec la composition comparative C.

### Evaluation du toucher des mèches.

On présente à un panel de 10 testeurs les mèches préparées comme indiqué ci-dessus.

On leur demande d'indiquer les mèches qu'ils jugent plus douces et lisses.

Les 10 juges sont unanimes et déclarent que les cheveux traités à l'aide de la composition A sont plus doux et lisses.

### Evaluation des propriétés fixantes

On procède de la même façon qu'à l'exemple 1.

La longueur des mèches suspendues s'accroît alors d'une certaine longueur (ΔL). Plus cet allongement ΔL est faible, meilleure est la tenue du coiffage dans le temps.

Ainsi, avec la composition C, l'allongement était de 3,6 cm (moyenne de 2 mèches), alors qu'il n'était que de 2,55 cm (moyenne de 2 mèches) pour la composition A, soit une amélioration dans la réduction de l'allongement de plus de 29%, ce qui traduit bien la meilleure tenue de coiffage obtenue grâce au shampooing selon l'invention.

### EXEMPLE 3

On a réalisé un shampooing conforme à l'invention de composition suivante :

| | |
|---|---|
| AKYPOSOFT 45 NV (KAO) | 12 g MA |
| AG 10 LK (KAO) | 2g MA |
| STYLEZE CC-10 (ISP) | 1 g MA |
| LUVISET PUR (BASF) | 1,23 g MA |
| Chlorure de sodium | 2 g |
| Eau qsp | 100 g |
| pH ajusté à | 7 |

### EXEMPLE 4

On a réalisé un shampooing conforme à l'invention de composition suivante :

| | |
|---|---|
| Lauryléther sulfate de sodium (2,2 OE) | 15,5 g MA |
| Cocoylbétaïne (DEHYTON AB 30 de HENKEL) | 2,4 g MA |
| JAGUAR C13 S (RHODIA) | 0,05 g MA |
| LUVISET PUR (BASF) | 1 g MA |
| Polydimethyl siloxane | 2,7 g MA |
| 1-(Hexadécyloxy) - 2 - octadécanol / alcool cétylique | 2,5 g MA |
| Monoisopropanolamide d'acides de coprah | 1 g MA |
| Cétostéaryl sulfate de sodium | 0,75 g MA |
| Conservateur q.s | |
| Eau q.s.p | 100 g |
| pH ajusté à | 7 |

### EXEMPLE 5

On a réalisé un shampooing conforme à l'invention de composition suivante :

| | |
|---|---|
| BEAULIGHT SHAA (SANYO KASEI) | 3 g MA |
| AG 10LK (KAO) | 12 g MA |
| MERQUAT 100 de CALGON | 1 g MA |
| LUVISET PUR (BASF) | 3 g MA |
| Chlorure de sodium | 1,5 g |
| Dioléate de méthyl glucoside oxyéthylène (120 OE) | 1 g MA |
| Eau q.s.p | 100 g |
| pH ajusté (NaOH/HCl) | 7 |

### EXEMPLE 6

On a réalisé un shampooing conforme à l'invention de composition suivante :

| | |
|---|---|
| BEAULIGHT SHAA ( SANYO KASEI) | 4,5 gMA |
| AG 10 LK (KAO) | 6 gMA |
| STYLEZE CC 10 (ISP) | 1 gMA |
| LUVISET PUR (BASF) | 4,05 gMA |
| Chlorure de sodium | 1,5 g |
| Dioleate de méthyl glucoside oxyethylène (120 OE) | 1,5 g |
| Eau q.s.p. | 100 |
| pH ajusté (NaOH / HCl) | 7 |

## Revendications

1. Composition détergente **caractérisée par le fait qu'**elle comprend, dans un milieu cosmétiquement acceptable, une base lavante, au moins un polymère cationique, au moins un polycondensat anionique comprenant au moins une séquence polyuréthanne et/ou polyurée, ledit polycondensat étant soluble dans l'eau.

2. Composition selon la revendication 1, **caractérisée par le fait que** ladite base lavante comprend un ou plusieurs tensioactifs choisis parmi des tensioactifs anioniques, amphotères, non ioniques, zwittérioniques et leurs mélanges.

3. Composition selon la revendication 1 ou 2, **caractérisée par le fait que** ladite base lavante est présente à une teneur pondérale comprise entre 4 % et 50 % par rapport au poids total de la composition, de préférence comprise entre 6 % et 25 %.

4. Composition selon l'une quelconque des revendications 1 à 3, **caractérisée par le fait que** ledit polycondensat est formé par un arrangement de blocs, cet arrangement étant obtenu à partir de:
(1) au moins un composé qui contient deux ou plus de deux atomes d'hydrogène actifs par molécule;
(2) au moins un diol ou un mélange de diols contenant des radicaux acides ou leurs sels;
(3) au moins un di- ou polyisocyanate.

5. Composition selon la revendication 4, **caractérisé par le fait que** les composés (1) sont choisis dans le groupe comprenant les diols, les diamines, les polyesterols, les polyétherols ou leur mélange.

6. Composition selon la revendication 4, **caractérisé par le fait que** le composé (2) est un acide carboxylique 2,2-hydroxyméthyl.

7. Composition selon la revendication 4, **caractérisé par le fait que** le composé (3) est choisi dans la groupe comprenant l'hexaméthylène diisocyanate, le toluylènediisocyanate, le diphénylméthane 4,4'-diisocyanate, le dicyclohexylméthane 4,4'-diisocyanate, le méthylène-di-p-phényl diisocyanate, le méthylène-bis(4-cyclohexylisocyanate), l'isophorone diisocyanate, le toluène diisocyanate, le 1,5-naphtalène diisocyanate, le 4,4'-diphénylméthane diisocyanate, le 2,2'-diméthyl-4,4'-diphénylméthane diisocyanate, le 1,3-phénylène diisocyanate, le 1,4-phénylène diisocyanate, des mélanges de 2,4- et de 2,6- toluène diisocyanate, le 2,2'-dichloro-4,4'-diisocyanato diphénylméthane, le 2,4-dibromo-1,5-diisocyanato naphtalène, le 1,4-diisocyanate butane, l'hexane-1,6-diisocyanate et le cyclohexane-1,4-diisocyanate.

8. Composition selon la revendication 4, **caractérisé par le fait que** le polycondensat est formé à partir d'au moins un composé supplémentaire ayant un squelette siliconé et choisi dans le groupe comprenant les polysiloxanes, les polyalkylsiloxanes ou les polyarylsiloxanes notamment les polyéthylsiloxanes, les polyméthylsiloxanes et les polyphénylsiloxanes, comportant éventuellement des chaînes hydrocarbonées greffées sur les atomes de silicium.

9. Composition selon l'une quelconque des revendications 1 à 3, **caractérisé par le fait que** les séquences de polyuréthanne et/ou polyurée du polymère présentent un motif répétitif de base répondant à la formule générale I' ci-après:
- X' - B - X' - CO - NH - R - NH - CO - (I')
dans laquelle :
- X' représente O et/ou NH,
- B est un radical hydrocarboné, ce radical étant substitué ou non, et
- R est un radical divalent choisi parmi les radicaux alkylène de type aromatique, aliphatique en C₁ à C₂₀, cycloaliphatique en C₁ à C₂₀, ces radicaux étant substitués
ou non.

10. Composition selon la revendication 9, **caractérisé par le fait que** B est un radical hydrocarboné bivalent en C₁ à C₃₀.

11. Composition selon la revendication 9 ou 10, **caractérisé par le fait que** le radical R est choisi dans le groupe comprenant les radicaux hexaméthylène, 4,4'-biphénylèneméthane, 2,4- et/ou 2,6-tolylène, 1,5-naphtylène, p-phénylène, méthylène-4,4bis - cyclohéxyle et le radical divalent dérivé de l'isophorone.

12. Composition selon l'une quelconque des revendications 1 à 3, **caractérisé par le fait que** le polycondensat présente un motif répétitif de base répondant à la formule (II'):
- X' - P - X' - CO - NH - R - NH - CO - (IIʹ)
dans laquelle :
- P est un segment polysiloxanique,
- X' représente O et/ou NH, et
- R est un radical divalent choisi parmi les radicaux alkylène de type aromatique, aliphatique en C₁ à C₂₀, cycloaliphatique en C₁ à C₂₀, ces radicaux étant substitués ou non.

13. Composition selon l'une quelconque des revendications précédentes, **caractérisée par le fait que** ledit polymère cationique est choisi parmi :
(1) les homopolymères ou copolymères dérivés d'esters ou d'amides acryliques ou méthacryliques et comportant au moins un des motifs de formules suivantes: 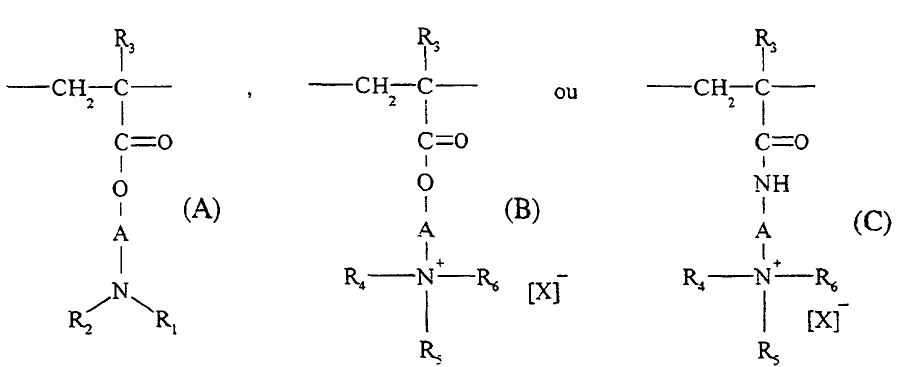 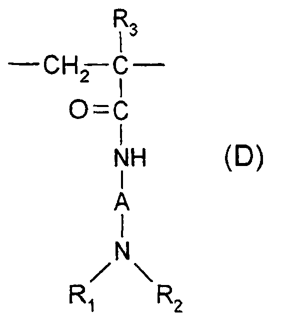 dans lesquelles:
R₃ , identiques ou différents, désignent un atome d'hydrogène ou un radical CH₃;
A, identiques ou différents, représentent un groupe alkyle, linéaire ou ramifié, de 1 à 6 atomes de carbone ou un groupe hydroxyalkyle de 1 à 4 atomes de carbone ;
R₄, R₅, R₆, identiques ou différents, représentent un groupe alkyle ayant de 1 à 18 atomes de carbone ou un radical benzyle;
R₁ et R₂ , identiques ou différents, représentent hydrogène ou un groupe alkyle ayant de 1 à 6 atomes de carbone;
X désigne un anion dérivé d'un acide minéral ou organique.
(2) Les dérivés d'éthers de cellulose comportant des groupements ammonium quaternaires,
(3) Les dérivés de cellulose cationiques tels que les copolymères de cellulose ou les dérivés de cellulose greffés avec un monomère hydrosoluble d'ammonium quaternaire,
(4) Les polysaccharides cationiques,
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères,
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées.
(7) les dérivés de polyaminoamides résultant de la condensation de polyalcoylènes polyamines avec des acides polycarboxyliques suivie d'une alcoylation par des agents bifonctionnels,
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone,
(9) les cyclopolymères d'alkyl diallyl amine ou de dialkyl diallyl ammonium
(10) le polymère de diammonium quaternaire contenant des motifs récurrents répondant à la formule : 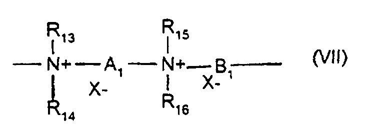 formule (VII) dans laquelle :
R13, R14, R15 et R16, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles contenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
dans lequel D désigne :
a) un reste de glycol de formule : -O-Z-O-, où Z désigne un radical hydrocarboné linéaire ou ramifié ou un groupement répondant à l'une des formules suivantes :
-(CH2-CH2-O)x-CH2-CH2-
-[CH2-CH(CH3)-O]y-CH2-CH(CH3)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent
-CH2-CH2-S-S-CH2-CH2- ;
d) un groupement uréylène de formule : -NH-CO-NH- ;
De préférence, X⁻ est un anion.
(11) les polymères de polyammonium quaternaires constitués de motifs de formule (VIII): 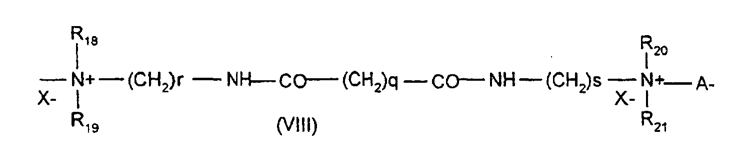 formule dans laquelle :
R18, R19, R20 et R21, identiques ou différents, représentent un atome d'hydrogène ou un radical méthyle, éthyle, propyle, β-hydroxyéthyle, β-hydroxypropyle ou -CH2CH2(OCH2CH2)pOH,
où p est égal à 0 ou à un nombre entier compris entre 1 et 6, sous réserve que R18, R19, R20 et R21 ne représentent pas simultanément un atome d'hydrogène,
r et s, identiques ou différents, sont des nombres entiers compris entre 1 et 6,
q est égal à 0 ou à un nombre entier compris entre 1 et 34,
X désigne un atome d'halogène,
A désigne un radical d'un dihalogénure ou représente de préférence -CH2-CH2-O-CH2-CH2-.
(12) Les polymères quaternaires de vinylpyrrolidone et de vinylimidazole,
(13) Les polyamines référencées sous le nom de « POLYETHYLENEGLYCOL (15) TALLOW POLYAMINE » dans le dictionnaire CTFA.
(14) Les polymères réticulés de sels de méthacryloyloxyalkyl(C₁-C₄) trialkyl(C₁₋C₄)ammonium,
(15) des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

14. Composition selon la revendication précédente, **caractérisée par le fait que** ledit polymère cationique est choisi parmi les dérivés d'éther de cellulose quaternaires, les cyclopolymères, les polysaccharides cationiques, les copolymères vinylpyrrolidone / méthacrylamidopropyl dimethylamine et leurs mélanges.

15. Compositions selon l'une quelconque des revendications précédentes, **caractérisées par le fait que** ledit polymère cationique est présent à une teneur pondérale comprise entre 0,01 % et 10 % par rapport au poids total de la composition et de préférence comprise entre 0,05 % et 5 %.

16. Compositions selon l'une quelconque des revendications précédentes, **caractérisées par le fait que** ledit polycondensat est présent à une teneur pondérale comprise entre 0,05 % et 10 % par rapport au poids total de la composition et de préférence comprise entre 0,1 % et 5 %.

17. Compositions selon l'une quelconque des revendications précédentes, **caractérisées par le fait qu'**elles présentent un pH compris entre 4 et 9.

18. Utilisation d'une composition telle définie à l'une quelconque des revendications précédentes pour le nettoyage et/ou le soin et/ou le conditionnement et/ou le coiffage des matières kératiniques.

19. Utilisation d'un polycondensat tel que défini à l'une quelconque des revendications précédentes pour améliorer l'effet coiffant d'une composition capillaire détergente contenant au moins un polymère cationique.

## Patentansprüche

1. Reinigende Zusammensetzung, **dadurch gekennzeichnet, dass** sie in einem kosmetisch akzeptablen Medium eine reinigende Basisformulierung, mindestens ein kationisches Polymer und mindestens ein anionisches Polykondensat enthält, das mindestens eine Polyurethansequenz und/oder Polyharnstoffsequenz aufweist, wobei das Polykondensat wasserlöslich ist.

2. Zusammensetzung nach Anspruch 1, **dadurch gekennzeichnet, dass** die reinigende Basisformulierung einen oder mehrere grenzflächenaktive Stoffe enthält, die unter den anionischen, amphoteren, nichtionischen und zwitterionischen grenzflächenaktiven Stoffen und deren Gemischen ausgewählt sind.

3. Zusammensetzung nach Anspruch 1 oder 2, **dadurch gekennzeichnet, dass** die reinigende Basisformulierung in einem Gewichtsanteil von 4 bis 50 %, bezogen auf das Gesamtgewicht der Zusammensetzung, und vorzugsweise 6 bis 25 % enthalten ist.

4. Zusammensetzung nach einem der Ansprüche 1 bis 3, **dadurch gekennzeichnet, dass** das Polykondensat aus einer Anordnung von Blöcken besteht, die erhalten wird ausgehend von:
(1) mindestens einer Verbindung, die zwei oder mehrere aktive Wasserstoffe pro Molekül enthält;
(2) mindestens einem Diol oder einem Gemisch von Diolen, die saure Gruppen oder deren Salze enthalten; und
(3) mindestens einem Di- oder Polyisocyanat.

5. Zusammensetzung nach Anspruch 4, **dadurch gekennzeichnet, dass** die Verbindungen (1) unter den Diolen, Diaminen, Polyesterolen, Polyetherolen oder deren Gemischen ausgewählt sind.

6. Zusammensetzung nach Anspruch 2, **dadurch gekennzeichnet, dass** die Verbindung (2) eine 2,2-Hydroxymethylcarbonsäure ist.

7. Zusammensetzung nach Anspruch 4, **dadurch gekennzeichnet, dass** die Verbindung (3) unter Hexamethylendiisocyanat, Toluylendiisocyanat, Diphenylmethan-4,4'-diisocyanat, Dicyclohexylmethan-4,4'-diisocyanat, Methylen-di-p-phenyldiisocyanat, Methylen-bis(4-cyclohexylisocyanat), Isophorondiisocyanat, Toluoldiisocyanat, 1,5-Naphthalindiisocyanat, 4,4'-Diphenylmethandiisocyanat, 2,2'-Dimethyl-4,4'-diphenylmethandiisocyanat, 1,3-Phenylendiisocyanat, 1,4-Phenylendiisocyanat, Gemischen von 2,4- und 2,6-Toluoldiisocyanat, 2,2'-Dichlor-4,4'-cliisocyanatodiphenylmethan, 2,4-Dibrom-1,5-diisocyanato-naphthalin, 1,4-Diisocyanatobutan, Hexan-1,6-diisocyanat und Cyclohexan-1,4-diisocyanat ausgewählt ist.

8. Zusammensetzung nach Anspruch 4, **dadurch gekennzeichnet, dass** das Polykondensat ausgehend von mindestens einer zusätzlichen Verbindung gebildet wird, welche ein Silicongerüst aufweist und unter den Polysiloxanen, Polyalkylsiloxanen oder Polyarylsiloxanen, insbesondere den Polyethylsiloxanen, Polymethylsiloxanen und Polyphenylsiloxanen, ausgewählt ist, die gegebenenfalls Kohlenwasserstoffketten enthalten, die auf die Siliciumatome gepfropft sind.

9. Zusammensetzung nach einem der Ansprüche 1 bis 3, **dadurch gekennzeichnet, dass** die Polyurethansequenzen und/oder Polyharnstoffsequenzen des Polymers eine wiederkehrende Grundeinheit der folgenden allgemeinen Formel (I') besitzen:
-X'-B-X'-CO-NH-R-NH-CO- (I'),
worin bedeuten:
- X' O und/oder NH,
- B eine zweiwertige Kohlenwasserstoffgruppe, wobei diese Gruppe unsubstituiert oder substituiert vorliegt, und
- R eine zweiwertige Gruppe, die unter den Alkylengruppen vom aromatischen Typ, vom aliphatischen Typ mit 1 bis 20 Kohlenstoffatomen und vom cycloaliphatischen Typ mit 1 bis 20 Kohlenstoffatomen ausgewählt ist, wobei diese Gruppen substituiert oder unsubstituiert sein können.

10. Zusammensetzung nach Anspruch 9, **dadurch gekennzeichnet, dass** die Gruppe B eine zweiwertige C₁₋₃₀-Kohlenwasserstoffgruppe ist.

11. Zusammensetzung nach Anspruch 9 oder 10, **dadurch gekennzeichnet, dass** die Gruppe R unter den Gruppen Hexamethylen, 4,4'-Biphenylenmethan, 2,4- und/oder 2,6-Toluylen, 1,5-Naphthylen, p-Phenylen, Methylen-4,4-bis-cyclohexyl und der von Isophoron abgeleiteten zweiwertigen Gruppe ausgewählt ist.

12. Zusammensetzung nach einem der Ansprüche 1 bis 3, **dadurch gekennzeichnet, dass** das Polykondensat eine wiederkehrende Grundeinheit der folgenden Formel (II') enthält:
-X'-P-X'-CO-NH-R-NH-CO- (II'),
worin bedeuten:
- P eine Polysiloxansegment,
- X' O und/oder NH,
- R eine zweiwertige Gruppe, die unter den Alkylengruppen vom aromatischen Typ, vom aliphatischen Typ mit 1 bis 20 Kohlenstoffatomen und vom cycloaliphatischen Typ mit 1 bis 20 Kohlenstoffatomen ausgewählt ist, wobei diese Gruppen substituiert oder unsubstituiert sein können.

13. Zusammensetzung nach einem der vorhergehenden Ansprüche, **dadurch gekennzeichnet, dass** das kationische Polymer ausgewählt ist unter:
(1) Homopolymeren oder Copolymeren, die von Acrylsäure- oder Methacrylsäureestern oder Acryl- oder Methacrylamiden abgeleitet sind und mindestens eine Einheit der folgenden Formeln enthalten: 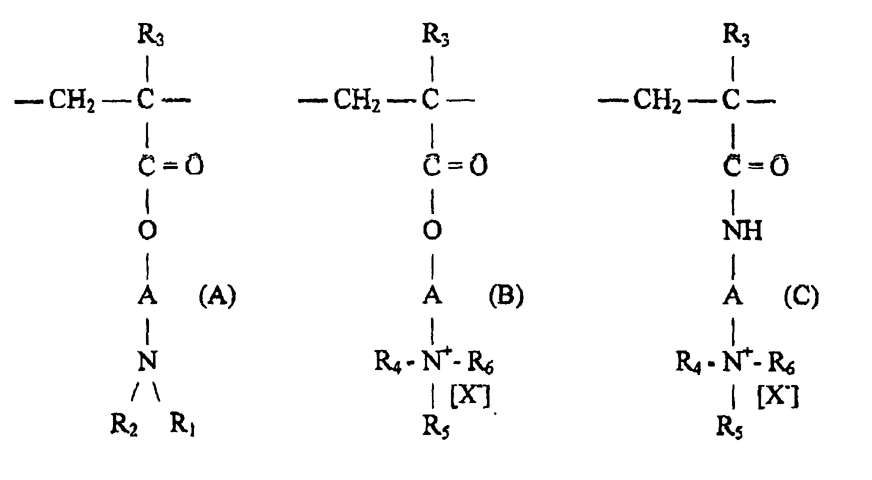 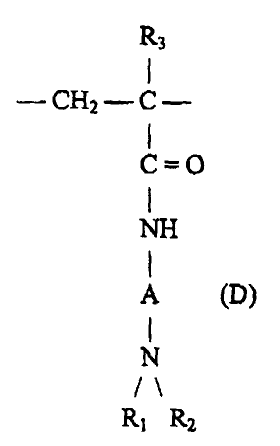 worin bedeuten:
die Gruppen R₃, die gleich oder verschieden sind, Wasserstoff oder die Gruppe CH₃;
die Gruppen A, die gleich oder verschieden sind, eine geradkettige oder verzweigte Alkylgruppe mit 1 bis 6 Kohlenstoffatomen oder eine Hydroxyalkylgruppe mit 1 bis 4 Kohlenstoffatomen;
die Gruppen R₄, R₅ und R₆, die identisch oder voneinander verschieden sind, eine Alkylgruppe mit 1 bis 18 Kohlenstoffatomen oder Benzyl;
die Gruppen R₁ und R₂, die gleich oder verschieden sind, Wasserstoff oder eine Alkylgruppe mit 1 bis 6 Kohlenstoffatomen; und
X ein Anion, das von einer anorganischen oder organischen Säure abgeleitet ist;
(2) Derivaten von Celluloseethern, die quartäre Ammoniumgruppen enthalten;
(3) Kationischen Cellulosederivaten, wie Cellulose-Copolymeren oder Cellulosederivaten, die mit einem wasserlöslichen quartären Ammoniummonomer gepfropft sind;
(4) Kationischen Polysacchariden;
(5) Polymeren, die aus Piperazinyl-Einheiten und zweiwertigen Alkylen- oder Hydroxyalkylen-Gruppen mit geraden oder verzweigten Ketten bestehen, die gegebenenfalls durch Sauerstoffatome, Schwefelatome oder Stickstoffatome oder aromatische oder heterocyclische Ringe unterbrochen sind, sowie den Oxidationsprodukten und/oder Quatemisierungsprodukten dieser Polymere;
(6) Wasserlöslichen Polyaminoamiden, die insbesondere durch Polykondensation einer Säure mit einem Polyamin hergestellt sind; diese Polyaminoamide können mit einem Epihalohydrin, einem Diepoxid, einem Dianhydrid, einem ungesättigten Dianhydrid, einem zweifach ungesättigten Derivat, einem Bis-halohydrin, einem Bis-azetidinium, einem Bis-haloacyldiamin, einem Alkyl-bis-halogenid oder auch einem Oligomer vernetzt sein, das bei der Umsetzung einer reaktiven bifunktionellen Verbindung und einem Bis-halohydrin, Bis-azetidinium, Bis-haloacyldiamin, Alkyl-bis-halogenid, Epihalohydrin, Diepoxid oder zweifach ungesättigten Derivat entsteht; das Vernetzungsmittel wird in Mengenanteilen von 0,025 bis 0,35 Mol pro Aminogruppe des Polyaminoamids eingesetzt. Die Polyaminoamide können alkyliert oder, wenn sie eine oder mehrere tertiäre Aminogruppen aufweisen, quaternisiert sein;
(7) Derivaten von Polyaminoamiden, die sich bei der Kondensation von Polyalkylenpolyaminen mit Polycarbonsäuren und anschließender Alkylierung mit bifunktionellen Mitteln ergeben;
(8) Polymeren, die durch Umsetzung eines Polyalkylenpolyamins, das zwei primäre Aminogruppen und mindestens eine sekundäre Aminogruppe aufweist, mit einer Dicarbonsäure hergestellt sind, die unter Diglykolsäure und gesättigten aliphatischen Dicarbonsäuren mit 3 bis 8 Kohlenstoffatomen ausgewählt ist;
(9) Alkyldiallylamin- oder Dialkyldiallylammonium-Cyclopolymeren;
(10) dem quartären Diammonium-Polymer mit wiederkehrenden Einheiten der folgenden Formel: 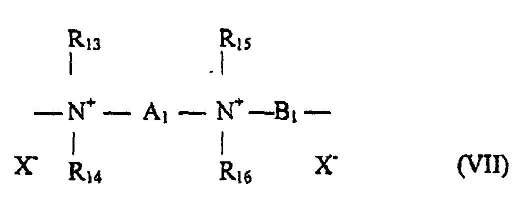 wobei in der Formel (VII) die Gruppen R₁₃, R₁₄, R₁₅ und R₁₆, die identisch oder voneinander verschieden sind, aliphatische, alicyclische oder arylaliphatische Gruppen mit 1 bis 20 Kohlenstoffatomen oder niedere hydroxyalkylaliphatische Gruppen bedeuten oder wobei die Gruppen R₁₃, R₁₄, R₁₅ und R₁₆ gemeinsam oder unabhängig voneinander mit den Stickstoffatomen, an die sie gebunden sind, Heterocyclen bilden, die gegebenenfalls ein zweites Heteroatom enthalten, das von Stickstoff verschieden ist, oder wobei die Gruppen R₁₃, R₁₄, R₁₅ und R₁₆ eine geradkettige oder verzweigte C₁₋₆-Alkylgruppe bedeuten, die mit einer Nitril-, Ester-, Acyl- oder Amidgruppe oder -CO-O-R₁₇-D oder -CO-NH-R₁₇-D substituiert ist, worin R₁₇ eine Alkylengruppe und D eine quartäre Ammoniumgruppe bedeuten;
A₁ und B₁ bedeuten Polymethylengruppen mit 2 bis 20 Kohlenstoffatomen, die geradkettig oder verzweigt, gesättigt oder ungesättigt sein können und die an die Hauptkette gebunden oder in der Hauptkette einen oder mehrere aromatische Ringe oder ein oder mehrere Sauerstoffatome oder Schwefelatome oder eine oder mehrere der folgenden Gruppen enthalten können: Sulfoxid, Sulfon, Disulfid, Amino, Alkylamino, Hydroxy, quartäre Ammoniumgruppen, Ureido, Amid oder Ester;
X⁻ bedeutet ein Anion, das von einer anorganischen oder organischen Säure abgeleitet ist;
Die Gruppen A₁, R₁₃ und R₁₅ können mit den beiden Stickstoffatomen, an die sie gebunden sind, einen Piperazinring bilden; wenn A₁ eine geradkettige oder verzweigte, gesättigte oder ungesättigte Alkylengruppe oder Hydroxyalkylengruppe bedeutet, kann die Gruppe B₁ auch eine Gruppe (CH₂)ₙ-CO-D-OC-(CH₂)ₙ- bedeuten, worin D bedeutet:
a) eine Glykolgruppe der Formel : -O-Z-O- , worin Z eine geradkettige oder verzweigte Kohlenwasserstoffgruppe oder eine Gruppe bedeutet, die einer der folgenden Formeln entspricht:
-(CH₂-CH₂-O)ₓ-CH₂-CH₂-
-[CH₂-CH(CH₃)-O]_{y}-CH₂-CH(CH₃)-
worin x und y eine ganze Zahl von 1 bis 4 bedeuten und einen wohl definierten und einzigen Polymerisationsgrad darstellen, oder eine beliebige Zahl von 1 bis 4 bedeuten und einen mittleren Polymerisationsgrad darstellen;
b) ein bis-sekundäres Diamin, beispielsweise ein Piperazinderivat,
c) ein bis-primäres Diamin der Formel : -NH-Y-NH- , worin Y eine geradkettige oder verzweigte Kohlenwasserstoffgruppe oder auch die zweiwertige Gruppe -CH₂-CH₂-S-S-CH₂-CH₂- bedeutet, oder
d) die Ureylengruppe der Formel : -NH-CO-NH-.
X⁻ bedeutet vorzugsweise ein Anion;
(11) Quartären Polyammonium-Polymeren, die aus Einheiten der folgenden Formel (VIII) bestehen: 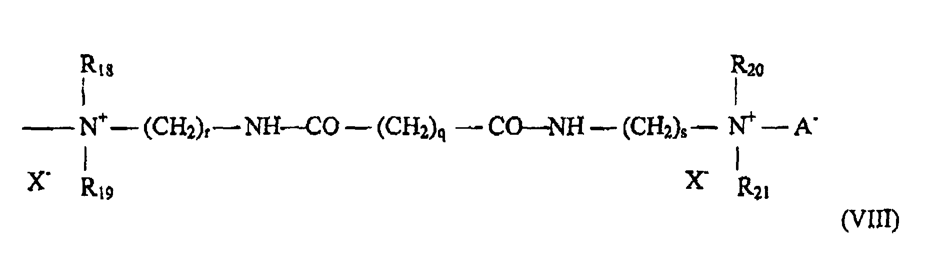 wobei in der Formel bedeuten:
R₁₈, R₁₉, R₂₀ und R₂₁, die identisch oder voneinander verschieden sind, ein Wasserstoffatom oder Methyl, Ethyl, Propyl, β-Hydroxyethyl, β-Hydroxypropyl oder -CH₂CH₂(OCH₂CH₂)ₚOH, wobei p Null oder eine ganze Zahl im Bereich von 1 bis 6 bedeutet, mit der Maßgabe, dass R₁₈, R₁₉, R₂₀ und R₂₁ nicht gleichzeitig Wasserstoff bedeuten,
r und s, die gleich oder verschieden sind, eine ganze Zahl
im Bereich von 1 bis 6,
q Null oder eine ganze Zahl im Bereich von etwa 1 bis 34,
X ein Halogenatom, und
A ein Dihalogenid oder vorzugsweise -CH₂CH₂-O-CH₂CH₂-.
(12) Quartären Polymeren von Vinylpyrrolidon und Vinylimidazol;
(13) Polyaminen, wie POLYQUART H, das von HENKEL im Handel ist und nach CTFA-Nomenklatur als 'POLYETHYLENGLYCOL (15) TALLOW POLYAMINE' bezeichnet wird;
(14) Vernetzten Polymeren von Methacryloyloxyalkyl(C₁₋₄)trialkyl(C₁₋₄)ammoniumsalzen;
(15) Polyalkyleniminen, insbesondere Polyethyleniminen, Polymeren, die Vinylpyridin- oder Vinylpyridinium-Einheiten enthalten, Kondensaten von Polyaminen und Epichlorhydrin, quartären Polyureylenen und Chitinderivaten.

14. Zusammensetzung nach den vorhergehenden Anspruch, **dadurch gekennzeichnet, dass** das kationische Polymer unter den quartären Cellulosederivaten, Cyclopolymeren, kationischen Polysacchariden, Vinylpyrrolidon/ Methacrylamidopropyldimethylamin-Copolymeren und deren Gemischen ausgewählt ist.

15. Zusammensetzung nach einem der vorhergehenden Ansprüche, **dadurch gekennzeichnet, dass** das kationische Polymer in einem Gewichtsanteil von 0,01 bis 10 %, bezogen auf das Gesamtgewicht der Zusammensetzung, und vorzugsweise 0,05 bis 5 % enthalten ist.

16. Zusammensetzung nach einem der vorhergehenden Ansprüche, **dadurch gekennzeichnet, dass** das Polykondensat in einem Gewichtsanteil von 0,05 bis 10 %, bezogen auf das Gesamtgewicht der Zusammensetzung, und vorzugsweise 0,1 bis 5 % enthalten ist.

17. Zusammensetzung nach einem der vorhergehenden Ansprüche, **dadurch gekennzeichnet, dass** sie eine pH-Wert im Bereich von 4 bis 9 aufweist.

18. Verwendung einer Zusammensetzung nach einem der vorhergehenden Ansprüche zur Reinigung und/oder zur Pflege und/oder zum Konditionieren und/oder für die Frisurgebung von keratinischen Materialien.

19. Verwendung eines Polykondensats nach einem der vorhergehenden Ansprüche zur Verbesserung der Frisurgebungseigenschaften einer reinigenden Zusammensetzung für die Haarbehandlung, die mindestens ein kationisches Polymer enthält.

## Claims

1. Detergent composition, **characterized in that** it comprises, in a cosmetically acceptable medium, a washing base, at least one cationic polymer, at least one anionic polycondensate comprising at least one polyurethane and/or polyurea sequence, the said polycondensate being water-soluble.

2. Composition according to Claim 1, **characterized in that** the said washing base comprises one or more surfactants chosen from anionic, amphoteric, nonionic and zwitterionic surfactants and mixtures thereof.

3. Composition according to Claim 1 or 2, **characterized in that** the said washing base is present in a weight content of between 4% and 50% relative to the total weight of the composition, preferably between 6% and 25%.

4. Composition according to any one of Claims 1 to 3, **characterized in that** the said polycondensate is formed by an arrangement of blocks, this arrangement being obtained from:
(1) at least one compound which contains two or more than two active hydrogen atoms per molecule;
(2) at least one diol or a mixture of diols containing acid radicals or salts thereof;
(3) at least one di- or polyisocyanate.

5. Composition according to Claim 4, **characterized in that** the compounds (1) are chosen from the group comprising diols, diamines, polyesterols, polyetherols or a mixture thereof.

6. Composition according to Claim 4, **characterized in that** the compound (2) is a 2,2-hydroxymethylcarboxylic acid.

7. Composition according to Claim 4, **characterized in that** the compound (3) is chosen from the group comprising hexamethylene diisocyanate, tolylene diisocyanate, diphenylmethane 4,4'-diisocyanate, dicyclohexylmethane 4,4'-diisocyanate, methylenebis(p-phenyl) diisocyanate, methylenebis(4-cyclohexyl isocyanate), isophorone diisocyanate, toluene diisocyanate, 1,5-naphthalene diisocyanate, 4,4'-diphenylmethane diisocyanate, 2,2'-dimethyl-4,4'-diphenylmethane diisocyanate, 1,3-phenylene diisocyanate, 1,4-phenylene diisocyanate, mixtures of 2,4- and 2,6-toluene diisocyanate, 2,2'-dichloro-4,4'-diisocyanatodiphenylmethane, 2,4-dibromo-1,5-diisocyanatonaphthalene, butane 2,4-diisocyanate, hexane 1,6-diisocyanate and cyclohexane 1,4-diisocyanate.

8. Composition according to Claim 4, **characterized in that** the polycondensate is formed from at least one additional compound having a silicone skeleton and chosen from the group comprising polysiloxanes, polyalkylsiloxanes or polyarylsiloxanes, in particular polyethylsiloxanes, polymethylsiloxanes and polyphenylsiloxanes, optionally comprising hydrocarbon-based chains grafted onto silicon atoms.

9. Composition according to any one of Claims 1 to 3, **characterized in that** the polyurethane and/or polyurea sequences of the polymer contain a base repeating unit corresponding to the general formula I' below:
-X'-B-X'-CO-NH-R-NH-CO- (I')
in which:
- X' represents O and/or NH,
- B is a hydrocarbon-based radical, this radical being substituted or unsubstituted, and
- R is a divalent radical chosen from alkylene radicals of aromatic, C₁ to C₂₀ aliphatic or C₁ to C₂₀ cycloaliphatic type, these radicals being substituted or unsubstituted.

10. Composition according to Claim 9, **characterized in that** B is a divalent C₁ to C₃₀ hydrocarbon-based radical.

11. Composition according to Claim 9 or 10, **characterized in that** the radical R is chosen from the group comprising hexamethylene, 4,4'-biphenylenemethane, 2,4- and/or 2,6-tolylene, 1,5-naphthylene, p-phenylene and methylene-4,4-bis(cyclohexyl) radicals and the divalent radical derived from isophorone.

12. Composition according to any one of Claims 1 to 3, **characterized in that** the polycondensate comprises a base repeating unit corresponding to formula (II'):
- X'-P-X'-CO-NH-R-NH-CO- (II')
in which:
- P is a polysiloxane segment,
- X' represents O and/or NH, and
- R is a divalent radical chosen from alkylene radicals of aromatic, C₁ to C₂₀ aliphatic or C₁ to C₂₀ cycloaliphatic type, these radicals being substituted or unsubstituted.

13. Composition according to any one of the preceding claims, **characterized in that** the said cationic polymer is chosen from:
(1) homopolymers or copolymers derived from acrylic or methacrylic esters or amides and comprising at least one of the units of the following formulae: 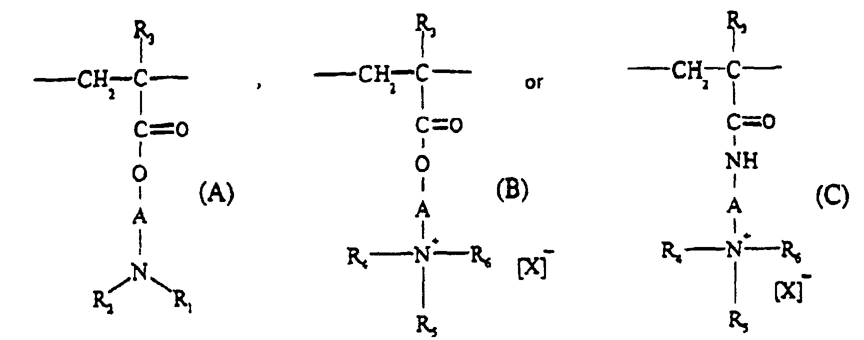 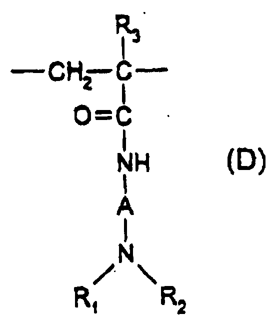 in which:
R₃, which may be identical or different, denote a hydrogen atom or a CH₃ radical;
A, which may be identical or different, represent a linear or branched alkyl group of 1 to 6 carbon atoms, or a hydroxyalkyl group of 1 to 4 carbon atoms;
R₄, R₅ and R₆, which may be identical or different, represent an alkyl group containing from 1 to 18 carbon atoms or a benzyl radical;
R₁ and R₂, which may be identical or different, represent hydrogen or an alkyl group containing from 1 to 6 carbon atoms;
X denotes an anion derived from an inorganic or organic acid,
(2) cellulose ether derivatives containing quaternary ammonium groups,
(3) cationic cellulose derivatives such as cellulose copolymers or cellulose derivatives grafted with a water-soluble monomer of quaternary ammonium,
(4) cationic polysaccharides,
(5) polymers consisting of piperazinyl units and of divalent alkylene or hydroxyalkylene radicals containing straight or branched chains, optionally interrupted by oxygen, sulphur or nitrogen atoms or by aromatic or heterocyclic rings, as well as the oxidation and/or quaternization products of these polymers,
(6) water-soluble polyamino amides prepared in particular by polycondensation of an acidic compound with a polyamine; these polyamino amides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent is used in proportions ranging from 0.025 to 0.35 mol per amine group of the polyamino amide; these polyamino amides can be alkylated or, if they contain one or more tertiary amine functions, they can be quaternized,
(7) the polyamino amide derivatives resulting from the condensation of polyalkylene polyamines with polycarboxylic acids followed by alkylation with difunctional agents,
(8) the polymers obtained by reaction of a polyalkylene polyamine containing two primary amine groups and at least one secondary amine group with a dicarboxylic acid chosen from diglycolic acid and saturated aliphatic dicarboxylic acids having from 3 to 8 carbon atoms,
(9) cyclopolymers of alkyldiallylamine or of dialkyldiallylammonium,
(10) the quaternary diammonium polymer containing repeating units corresponding to the formula: 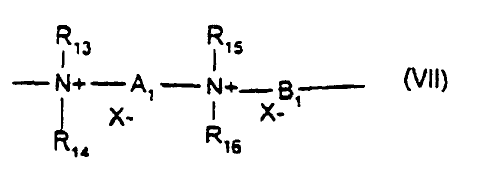 in which formula (VII):
R₁₃, R₁₄, R₁₅ and R₁₆, which may be identical or different, represent aliphatic, alicyclic or arylaliphatic radicals containing from 1 to 20 carbon atoms or lower hydroxyalkylaliphatic radicals, or alternatively R₁₃, R₁₄, R₁₅ and R₁₆, together or separately, constitute, with the nitrogen atoms to which they are attached, heterocycles optionally containing a second heteroatom other than nitrogen, or alternatively R₁₃, R₁₄, R₁₅ and R₁₆ represent a linear or branched C₁-C₆ alkyl radical substituted with a nitrile, ester, acyl or amide group or a group -CO-O-R₁₇-D or -CO-NH-R₁₇-D where R₁₇ is an alkylene and D is a quaternary ammonium group;
A₁ and B₁ represent polymethylene groups containing from 2 to 20 carbon atoms which may be linear or branched, saturated or unsaturated, and which may contain, linked to or intercalated in the main chain, one or more aromatic rings or one or more oxygen or sulphur atoms or sulphoxide, sulphone, disulphide, amino, alkylamino, hydroxyl, quaternary ammonium, ureido, amide or ester groups, and
X⁻ denotes an anion derived from an inorganic or organic acid;
A₁, R₁₃ and R₁₅ can form, with the two nitrogen atoms to which they are attached, a piperazine ring; in addition, if A₁ denotes a linear or branched, saturated or unsaturated alkylene or hydroxyalkylene radical, B₁ can also denote a group (CH₂)ₙ-CO-D-OC-(CH₂)ₙ-
in which D denotes:
a) a glycol residue of formula: -O-Z-O-, where Z denotes a linear or branched hydrocarbon radical or a group corresponding to one of the following formulae:
- (CH₂-CH₂-O)ₓ-CH₂-CH₂-
- [CH₂-CH(CH₃)-O]_{y}-CH₂-CH(CH₃)-
where x and y denote an integer from 1 to 4, representing a defined and unique degree of polymerization or any number from 1 to 4 representing an average degree of polymerization;
b) a bis-secondary diamine residue such as a piperazine derivative;
c) a bis-primary diamine residue of formula: -NH-Y-NH-, where Y denotes a linear or branched hydrocarbon radical, or alternatively the divalent radical
-CH₂-CH₂-S-S-CH₂-CH₂-;
d) a ureylene group of formula: -NH-CO-NH-; preferably, X⁻ is an anion,
(11) quaternary polyammonium polymers consisting of units of formula (VIII): 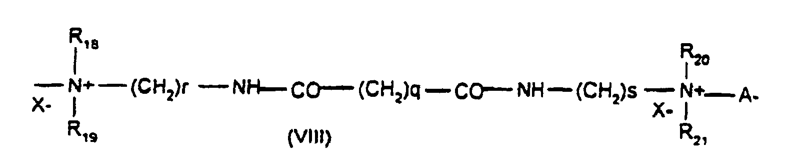 in which formula:
R₁₈, R₁₉, R₂₀ and R₂₁, which may be identical or different, represent a hydrogen atom or a methyl, ethyl, propyl, β-hydroxyethyl, β-hydroxypropyl or -CH₂CH₂(OCH₂CH₂)ₚOH radical,
where p is equal to 0 or to an integer between 1 and 6, with the proviso that R₁₈, R₁₉, R₂₀ and R₂₁ do not simultaneously represent a hydrogen atom,
r and s, which may be identical or different, are integers between 1 and 6,
q is equal to 0 or to an integer between 1 and 34,
X denotes a halogen atom,
A denotes a dihalide radical or preferably represents -CH₂-CH₂-O-CH₂-CH₂- ,
(12) quaternary polymers of vinylpyrrolidone and of vinylimidazole,
(13) the polyamines under the reference name "Polyethylene glycol (15) tallow polyamine" in the CTFA dictionary,
(14) crosslinked polymers of methacryloyloxy(C₁-C₄)alkyltri(C₁-C₄)-alkylammonium salts,
(15) polyalkyleneimines, in particular polyethyleneimines, polymers containing vinylpyridine or vinylpyridinium units, condensates of polyamines and of epichlorohydrin, quaternary polyureylenes and chitin derivatives.

14. Composition according to the preceding claim, **characterized in that** the said cationic polymer is chosen from quaternary cellulose ether derivatives, cyclopolymers, cationic polysaccharides, vinylpyrrolidone/methacrylamidopropyldimethylamine copolymers and mixtures thereof.

15. Compositions according to any one of the preceding claims, **characterized in that** the said cationic polymer is present in a weight content of between 0.01% and 10% relative to the total weight of the composition, and preferably between 0.05% and 5%.

16. Compositions according to any one of the preceding claims, **characterized in that** the said polycondensate is present in a weight content of between 0.05% and 10% relative to the total weight of the composition, and preferably between 0.1% and 5%.

17. Compositions according to any one of the preceding claims, **characterized in that** they have a pH of between 4 and 9.

18. Use of a composition as defined in any one of the preceding claims, for cleansing and/or caring for and/or conditioning and/or styling keratin substances.

19. Use of a polycondensate as defined in any one of the preceding claims, for improving the styling effect of a detergent hair composition containing at least one cationic polymer.
